(19) Europäisches Patentamt
European Patent Office
Office européen des brevets

(11) Publication number: 0 132 543
B1

# (12) EUROPEAN PATENT SPECIFICATION

(45) Date of publication of the patent specification: 08.10.86

(51) Int. Cl.[4]: C 07 C 17/00

(21) Application number: 84106220.1

(22) Date of filing: 30.05.84

## (54) Isomerization of allylic halides with quaternary salts.

(30) Priority: 20.07.83 US 515564

(43) Date of publication of application: 13.02.85 Bulletin 85/7

(45) Publication of the grant of the patent: 08.10.86 Bulletin 86/41

(84) Designated Contracting States: BE CH DE FR GB LI NL

(56) References cited:
US - A - 3 819 730
US - A - 3 836 592

(73) Proprietor: Union Camp Corporation, 1600 Valley Road, Wayne New Jersey 07470 (US)

(72) Inventor: Mitchell, Peter W.D., 106 Lancaster Road, Freehold, NJ 07728 (US)
Inventor: McElligott, Lois T., 1158 Wheatsheaf Lane, Abington, PA 19001 (US)
Inventor: Sasser, David E., 6290 Nancy Drive, Jacksonville, FA 32210 (US)

(74) Representative: von Füner, Alexander, Dr. et al, Patentanwälte v. Füner, Ebbinghaus, Finck Mariahilfplatz 2 & 3, D-8000 München 90 (DE)

EP 0 132 543 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### *BACKGROUND OF THE INVENTION*

*Field of the Invention*

The invention relates to the isomerization of allylic halides.

*Brief Description of the Prior Art*

Representative of the prior art is the description given in the U.S. Patent 3,819,730. In spite of the availability of several known methods of isomerizing allylic halides, there has remained a need for economical processes, saving of energy. The method of the present invention is such an improvement over the prior art, requiring low temperatures and short isomerization times.

### *SUMMARY OF THE INVENTION*

The invention comprises in a method for isomerizing an allylic halide to its allylic isomer which comprises isomerizing the allylic halide in the presence of a catalytic proportion of a hydrogen halide and a copper catalyst, the improvement which comprises; carrying out the isomerization in the presence of an organic quaternary salt selected from those of the formula:

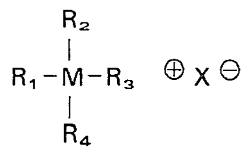

(I)

wherein X is selected from the group consisting of an organic and inorganic anion such as nitrate, benzoate, phenylacetate, hydroxybenzoate, phenoxide, hydroxide, cyanide, nitrite; particularly preferred are chloride, bromide, iodide, methyl sulfate, ethyl sulfate and the like; M represents nitrogen, arsenic, or phosphorus. $R_1$, $R_2$, $R_3$, and $R_4$ are each independently selected from one of those groups consisting of hydrocarbyl and substituted hydrocarbyl provided that when one or both of $R_1$ and $R_2$ contain from 1 to 4 carbon atoms, inclusive, the remainder of R moities will each contain from 6 to 25 carbon atoms, or $R_1$ and $R_2$ may be taken together to represent a divalent moiety attached to the atom M, and which is selected from the group consisting of alkenylene and hydrocarbyl-substituted alkenylene having 5 to 10 carbon atoms, inclusive, in the ring thereof; or $R_1$ and $R_2$ may be taken together with the atom of M to which they are attached to represent a divalent or monovalent moiety selected from the groups consisting of those having the formula:

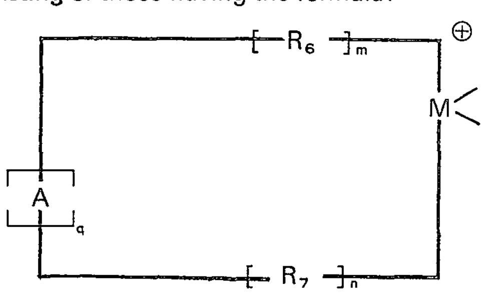

wherein A represents nitrogen, oxygen, sulfur, phosphorus and the like; and $R_6$ and $R_7$ are each selected from alkenylene and hydrocarbyl-substituted alkenylene of 1 to 25 carbon atoms, inclusive, m, n and q are each integers of 0 to 1 and the sum of m + n is 1 or 2.

The term «halide» as used herein is embracive of chloride, bromide and iodide.

The term «hydrocarbyl» as used herein means the monovalent moiety obtained upon removal of a hydrogen atom from a parent hydrocarbon. Representative of hydrocarbyl are alkyl of 1 to 25 carbon atoms, inclusive, such as methyl, ethyl, propyl, butyl, pentyl, hexyl, heptyl, octyl, nonyl, undecyl, decyl, dodecyl, octadecyl, nonodecyl, eicosyl, heneicosyl, docosyl, tricosyl, tetracosyl, pentacosyl and the isomeric forms thereof; aryl of 6 to 25 carbon atoms, inclusive, such as phenyl, tolyl, xylyl, naphthyl, biphenyl, tetraphenyl and the like; aralkyl of 7 to 25 carbon atoms, inclusive, such as benzyl, phenethyl, phenpropyl, phenbutyl, phenhexyl, naphthoctyl and the like; cycloalkyl of 3 to 8 carbon atoms, inclusive, such as cyclopropyl, cyclobutyl, cyclopentyl, cyclohexyl, cycloheptyl, cyclooctyl and the like; alkenyl of 2 to 25 carbon atoms, inclusive, such as vinyl, allyl, butenyl, pentenyl, hexenyl, octenyl, nonenyl, decenyl, undecenyl, dodecenyl, tridecenyl, pentadecenyl, octadecenyl, pentacosynyl and isomeric forms thereof.

The term «alkenylene» means the divalent moiety obtained on removal of two hydrogen atoms, each from a non-adjacent carbon atom of a parent hydrocarbon and includes alkenylene of 3 to 10 carbon atoms, inclusive, such as 1,3-propenylene, 1,4-butenylene, 1,5-pentenylene, 1,8-octenylene, 1,10-decenylene and the like.

The terms «substituted hydrocarbyl» and «substituted alkenylene» as used herein mean the hydrocarbyl or alkenylene moiety as previously defined wherein one or more hydrogen atoms have been replaced with an inert group, i.e. a chemical group which does not adversely affect the desired function of the organic quaternary salt of formula (I). Representative of such groups are aminophosphino-, hydrocarbyl, quaternary nitrogen (ammonium), quaternary phosphorus (phosphonium), hydroxyl-, alkoxy, mercapto-, alkyl, halo-, phosphate, phosphite, carboxylate groups and the like.

It is preferred to use compounds wherein the halogen is chloride.

The process of the invention is especially carried out at a temperature of 0° C to 50° C.

A further preferred embodiment of the invention is the use of cuprous chloride as copper catalyst.

It is furthermore preferred to use a copper catalyst within the weight range of 0.01 to 2.0% of allylic halide whereby the molar ratio of organic quaternary salt to copper catalyst should be 0.01 to 5.0.

It is preferred to carry out the isomerization for a period of 0.1 to 10.0 hours.

Especially preferred is the use of a mixture of tri(alkyl)methyl ammonium chlorides where the

alkyl portion comprises a chain of from eight to ten carbons as the organic quaternary salt.

Especially preferred are furthermore compounds selected from the group consisting of methyltrioctylammonium chloride, dimethylbenzylstearyl ammonium, N,N-cetylethyl-morpholinium ethosulfate, benzyl hydroxyethyl(2) coco-imidazolium chloride, N-tallow penta methyl propane diammoniumchloride, benzyltrioctyl ammonium bromide and propyltriphenyl phosphonium bromide.

As later on discussed the process of the invention may be carried out especially for isomerizing linalyl chloride or 3-chloro-3-methyl-1-butene.

*DETAILED DESCRIPTION OF THE PREFERRED EMBODIMENT OF THE INVENTION*

The method of the invention may be employed to isomerize any allylic halide to obtain its allylic isomer. In other words, a tertiary allylic halide may be isomerized to the corresponding secondary or primary allylic halide and the secondary allylic halide may be isomerized to the corresponding primary allylic halide. Representative of an advantageous isomerization according to the method of the invention is the isomerization of linalyl chloride to the geranyl and neryl chlorides according to the schematic formulae:

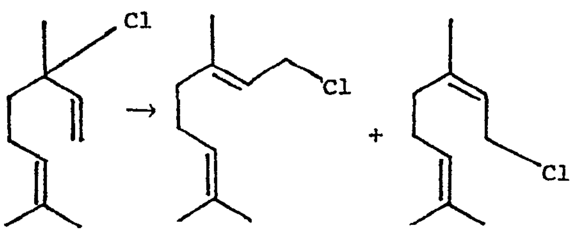

linalyl chloride geranyl chloride neryl chloride

The method of the invention is not limited to the isomerization of linalyl chloride to geranyl and neryl chlorides but may be used to isomerize any allylic halide to its less allylic isomer.

Other representative allylic halides which may be advantageously isomerized include 3,4-dichloro-1-butene and especially 3-chloro-3-methyl-1-butene and like allylic halides.

The method of the invention is an improvement over isomerizations carried out in the presence of hydrogen halides and copper catalysts. Representative of hydrogen halides so employed are hydrogen chloride and hydrogen bromide. In general, catalytic proportions of the hydrogen halide are used. Catalytic proportions are generally within the range of from about 0.01 to 5 percent by weight of the starting halides, preferably 0.1 to 1.0 percent.

The copper catalysts employed may be any copper compound having a valency of 2 or less, including metallic copper. Any copper compound convertible to the halide such as the bromide, iodide or chloride under conditions of the reaction may also be used. Representative of copper catalysts advantageously employed are the chloride, bromide, carbonate, oxide, acetate, formate, sulfate and like derivative cupric and cuprous compounds. Preferred as the copper catalyst in the improved process of the invention is cuprous chloride. Catalytic proportions of the copper catalyst are generally within the weight range of from about 0.01 to 2 percent of the allylic halide starting compound, preferably about 0.5 percent.

Organic quaternary compounds of the formula (I) given above are generally well known as are methods of their preparation. Representative of such organic quaternary compounds are trioctylmethylammonium chloride, tetraoctadecylammonium chloride, dodecyldimethylbenzyl-ammonium chloride, tetradecyldimethylbenzylammonium chloride, hexadecyldimethylbenzylammonium chloride, N,N-cetylethylmorpholinium ethosulfate, methyl(1)cocoamido-ethyl(2)cocoimidazolinium methyl sulfate, N-tallow-pentamethylpropanediammonium dichloride, trioctylmethyl-phosphonium bromide, N,N-soya ethylmorpholinium ethosulfate, hexadecylpyridinium chloride, benzyl hydroxyethyl(2)-cocoimidazolinium chloride, dodecyldimethyl(ethylbenzyl)-ammonium chloride, tetradecyldimethyl(ethylbenzyl)ammonium chloride, hexadecyldimethyl-(ethylbenzyl)ammonium chloride, octadecyl-dimethyl(ethylbenzyl)ammonium chloride, octadecyldimethylbenzylammonium chloride, methyl bis (2-hydroxyethyl)cocoammonium chloride, methyl(1)soyaamido-ethyl(2)soyaimidazolinium methyl sulfate and the like.

Commercially available quaternary salts or purified forms of quaternary salts may be used in the preferred process of the invention.

It will be appreciated that under specific conditions of operating the process of the invention, certain of the above described compounds of the formula (I) given above have advantages over other compounds of the same general formula. Selection of a particular compound (I) for use under specific process conditions for optimum yields may be made by trial and error techniques. We have observed however that there are advantages associated with a mixture of trialkylmethylammonium chlorides where the alkyl portion consists of a chain of from eight to ten carbon atoms. For example, Adogen 464 (Wherex Chemical Co.).

The organic quaternary salt is used in a proportion to isomerize at least some of the allylic halide according to the method of the invention. Such a proportion is generally within the range of from about 0.01 to 10 percent by weight of the halide charge, preferably 0.2 to 2.5 percent.

Optimum proportions will depend to some extend upon the salt selected and may be determined by trial and error technique. Generally the preferred molar ratio of compound (I) to copper catalyst is from 0.01 to 5.0.

The method of the invention may be carried out by admixing the starting allylic halide with the hydrogen halide, copper catalyst and salt compound of the formula (I) in a suitable reaction vessel for a sufficient period of time to effect the desired isomerization. The controlling reaction rate in the method of the invention is the isomerization of the allylic halide to the desired allylic isomer.

This is controlled by residence time in the reaction zone. We have found that in isomerization of linalyl chloride the preferred minimum total residence time is within the range of from 0.1 to 10 hours and most preferably 0.5 to 5 hours under preferred operating temperatures.

Although the method of the invention may be carried out under a wide range of operating temperatures, i.e. within the range of from about 0° C. to 50° C., it is preferred to do so at a temperature of from 0° C to 30° C. and most preferably 0° C. to 20° C.

The method of the invention is not dependent upon pressure, and may be carried out at atmospheric, sub-atmospheric or super-atmospheric pressures.

Progress of the isomerization may be monitored by conventional analytical techniques. When it has been determined that isomerization occurred to a maximum desired point, the product mixture may be passed from the reaction apparatus. The desired allylic halide isomer may then be separated from the reaction mixture employing conventional and known techniques including washing, decantation, distillation and like techniques.

The following examples describe the manner and process of making and using the invention and set forth the best mode contemplated by the inventors of carrying out the invention but are not to be construed as limiting. All parts given are by weight unless otherwise indicated.

*Example 1*

To 8.0g of myrcene hydrochlorides, consisting of 37.0 parts linalyl chloride, 9.5 parts neryl chloride, 7.0 parts geranyl chloride, 5.0 parts *alpha*-terpinyl chloride and 41.5 parts hydrocarbons, was added 0.03g cuprous chloride, 0.11g Adogen 464* (a mixture of trialkylmethylammonium chlorides where the alkyl groups comprise 44% of $C_{10}$, 52% of $C_8$, 2% of $C_6$ and 2% of $C_{12}$) and 0.02g of hydrogen chloride gas. The mixture was stirred at 10° C. for 7 hours. Samples were withdrawn at 1-2 hour intervals, neutralized with aqueous sodium hydroxide, and analyzed by gas chromatography. Product content of linalyl, neryl and geranyl chloride (abbreviated LC1, NC1 and GC1 respectively) are shown below. The amount of time required to reduce the LC1 content by one-half was 1 hour.

| Time (Hrs) | Composition of Product (%) | | |
|---|---|---|---|
| | LC1 | NC1 | GC1 |
| 0 | 37.0 | 9.5 | 7.0 |
| 1.5 | 10.5 | 16.4 | 23.5 |
| 3.0 | 7.2 | 17.2 | 25.5 |
| 6.5 | 5.1 | 17.5 | 26.8 |

*Examples 2-11*

Myrcene hydrochlorides were subjected to isomerization under the same conditions and amounts as described in Example 1 using 0.03g cuprous chloride, 0.02g hydrogen chloride gas, and an organic quaternary salt as described in Table 1 to obtain the results shown in Table 1 below. The Examples 10 and 11 are not of the invention but are made for comparative purposes.

*Table 1*

| Example No. | Quaternary Salt And Amount Used | Amount of time to Reduce LC1 Content By One-half (hrs) | GC1:NC1 Ratio After 6.5 hours |
|---|---|---|---|
| 2 | None | 5-6 | 1.4 |
| 3 | Methyltrioctylammonium chloride, 0.11 g | 1 | 1.4 |
| 4 | Dimethylbenzylstearyl ammonium chloride, 0.11 g | 1 | 1.6 |
| 5 | N,N-cetylethylmorpholinium ethosulfate, 0.12 g | 1 | 1.7 |
| 6 | Benzyl hydroxyethyl (2) cocoimidazolium chloride, 0,12 g | 1 | 1.8 |
| 7 | N-Tallowpentamethyl propane diammonium dichloride, 0.12 g | 1 | 1.9 |
| 8 | Benzyltrioctylammonium bromide, 0,14 g | 2 | 1.4 |
| 9 | Propyltriphenyl phosphonium bromide, 0,15 g | 3 | 1.4 |
| 10 | Trimethylbenzylammonium chloride, 0.05 gm | >10 | 0.9 |
| 11 | Trimethylbutylammonium bromide, 0.05 gm | >10 | 0.8 |

*Example 12*

To 6.0g of isoprene hydrochlorides consisting of 63 parts 3-chloro-3-methyl-1-butene (abbreviated 3,3,1-CMB) and 37 parts 1-chloro-3-methyl-2-butene (abbreviated 1,3,2-CMB) was added 0.03g cuprous chloride, 0.02g hydrogen chloride gas and 0.15 gms of benzylhydroxyethyl(2) coco imidazolinium chloride. The mixture was stirred at 10° C. for 2 hours. A sample was withdrawn after 2 hours, neutralized with aqueous sodium hydroxide, and analyzed by nuclear magnetic resonance spectroscopy. The results obtained are shown in Table 2, below.

*Table 2*

| Hours | Product Composition (%) | |
|---|---|---|
| | 3,3,1-CMB | 1,3,2-CMB |
| 0 | 63 | 37 |
| 2 | 11 | 89 |

* A mixture of trialkylmethylammonium chlorides where the alkyl groups comprise 44% of $C_{10}$, 52% of $C_8$, 2% of $C_6$ and 2% of $C_{12}$.

*Example 13*

To 4.0g of dichlorobutene mixture consisting of 97 parts 3,4-dichloro-1 butene and 3 parts 1,4-dichloro-2-butene was added 0.02g cuprous chloride, an organic quaternary salt as described in Table 3, below, and 0.01g hydrogen chloride gas. The mixture was stirred at 50° C. for 5 hours, neutralized with aqueous sodium hydroxide and analyzed by gas chromatography. 3,4-dichloro-1-butene and 1,4-dichloro-2-butene are abbreviated 3,4-DCB and 1,4-DCB, respectively.

*Table 3*

| Quaternary Salt And Amount Added | Composition of Product (%) | | |
|---|---|---|---|
| | 3,4-DCB | Cis-1,4-DCB | trans-1,4-DCB |
| None | 91 | 1 | 8 |
| Adogen 464*, 0.009 g | 48 | 1 | 51 |
| Trimethylbenzylammonium chloride**, 0.04 g | 67 | 1 | 32 |

* Sherex Chemical Co.; A mixture of tri(alkyl)methylammonium chlorides where the alkyl portion is a chain of from 8 to 10 carbon atoms.

** Not a compound of the formula (I) for control purposes.

## Claims

1. In a method for isomerizing an allylic halide to its allylic isomer which comprises isomerizing the allylic halide in the presence of a catalytic proportion of a hydrogen halide and a copper catalyst, the improvement which comprises; carrying out the isomerization in the presence of an anorganic quaternary salt selected from those of the formula:

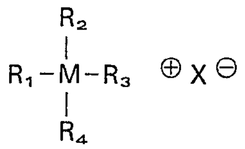

wherein X is selected from the group consisting of organic and inorganic anions such as nitrate, benzoate, phenylacetate, hydroxybenzoate, phenoxide, hydroxide, cyanide, nitrite; particularly preferred are chloride, bromide, iodide, methyl sulfate, ethyl sulfate and the like; M represents nitrogen, arsenic, or phosphorus. $R_1$, $R_2$, $R_3$, and $R_4$ are each independently selected from one of those groups consisting of hydrocarbyl and substituted hydrocarbyl provided that when one or both of $R_1$ and $R_2$ contain from 1 to 4 carbon atoms, inclusive, the remainder of R moieties will each contain from 6 to 25 carbon atoms, or $R_1$ and $R_2$ may be taken to represent a divalent moiety attached to the atom M, and which is selected from the group consisting of alkenylene and hydrocarbyl-substituted alkenylene having 5 to 10 carbon atoms, inclusive, in the ring thereof; or $R_1$ and $R_2$ may be taken together with the atom of M to which they are attached to represent a divalent or monovalent moiety selected from the groups consisting of those having the formula:

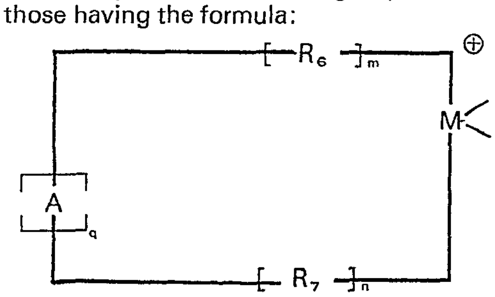

wherein A represents nitrogen, oxygen, sulfur, phosphorus and the like; and $R_6$ and $R_7$ are each selected from alkenylene and hydrocarbyl-substituted alkenylene of 1 to 25 carbon atoms, inclusive; m, n and q are each integers of 0 to 1 and the sum of m + n is 1 or 2.

2. The process of claim 1 wherein the halogen is chlorine.

3. The process of claim 1 or 2 carried out at a temperature of 0° C to 50° C.

4. The process of one of the claims 1-3 wherein the copper catalyst is cuprous chloride.

5. The process of one of the claims 1-4 wherein the catalytic proportion of copper catalyst is within the weight range of 0.01 to 2.0% of allylic halide.

6. The process of one of the claims 1-5 wherein the molar ratio of organic quaternary salt to copper catalyst is 0.01 to 5.0.

7. The process of one of the claims 1-6 wherein the isomerization is carried out for a period of 0.1 to 10.0 hours.

8. The process of one of the claims 1-7 wherein the organic quaternary salt is a mixture of tri(alkyl)methyl ammonium chlorides where the alkyl portion comprises a chain of from eight to ten carbons.

9. The process of one of the claims 1-8 wherein the organic quaternary salt is selected from a group consisting of methyltrioctylammonium chloride, dimethylbenzylstearyl ammonium, N,N-cetylethylmorpholinium ethosulfate, benzyl hydroxyethyl(2) cocoimidazolium chloride, N-tallow penta methyl propane diammoniumdichloride, benzyltrioctyl ammonium bromide and propyltriphenyl phosphonium bromide.

10. The process of one of the claims 1-9 wherein the allylic halide is linalyl chloride.

11. The process of one of the claims 1-9 wherein the allylic halide is 3-chloro-methyl-1-butene.

## Patentansprüche

1. Verfahren zur Isomerisierung eines Allylhalogenids zu dessen Allylisomer, wobei das Verfahren

die Isomerisierung des Allylhalogenids in Gegenwart einer katalytischen Menge eines Halogenwasserstoffs und eines Kupferkatalysators umfasst, und die Verbesserung des Verfahrens darin besteht, dass die Isomerisierung durchgeführt wird in Gegenwart eines organischen quaternären Salzes, ausgewählt aus solchen der Formel

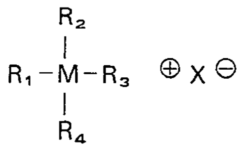

worin X ausgewählt ist aus der Gruppe, bestehend aus organischen und anorganischen Anionen wie Nitrat, Benzoat, Phenylacetat, Hydroxybenzoat, Phenoxid, Hydroxid, Cyanid, Nitrit, wobei Chlorid, Bromid, Jodid, Methylsulfat, Ethylsulfat etc. besonders bevorzugt sind, M für Stickstoff, Arsen oder Phosphor steht und worin $R_1$, $R_2$, $R_3$ und $R_4$ entweder jeweils unabhängig ausgewählt sind aus einer der Gruppen, bestehend aus Hydrocarbyl und substituiertem Hydrocarbyl, mit der Massgabe, dass dann, wenn entweder $R_1$ oder $R_2$ oder $R_1$ und $R_2$ 1 bis 4 Kohlenstoffatome enthalten, die restlichen Gruppen R jeweils von 6 bis 25 Kohlenstoffatome enthalten, oder $R_1$ und $R_2$ jeweils eine mit dem M-Atom verbundene zweiwertige Gruppe bedeuten, die ausgewählt ist aus der Gruppe, bestehend aus Alkenylen und hydrocarbylsubstituiertem Alkenylen mit 5 bis 10 Kohlenstoffatomen im Ring, oder $R_1$ und $R_2$ zusammen mit dem ihnen verbundenen M-Atom eine zwei- oder einwertige Gruppe bedeuten, ausgewählt aus den Gruppen, bestehend aus solchen der Formel

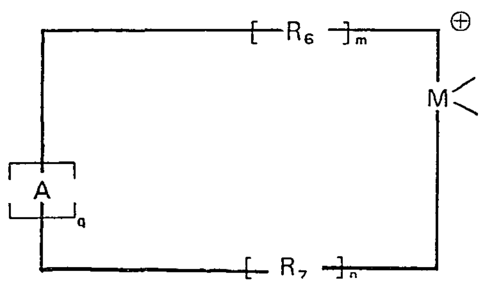

worin A Stickstoff, Sauerstoff, Schwefel, Phosphor und dergleichen bedeutet, $R_6$ und $R_7$ jeweils ausgewählt sind aus Alkenylen und hydrocarbylsubstituiertem Alkenylen mit von 1 bis 25 Kohlenstoffatomen, m, n und q jeweils ganze Zahlen von 0 bis 1 sind und die Summe von m + n 1 oder 2 ist.

2. Verfahren nach Anspruch 1, worin das Halogen Chlor ist.

3. Verfahren nach Anspruch 1 oder 2, das bei einer Temperatur von 0 bis 50° C durchgeführt wird.

4. Verfahren nach einem der Ansprüche 1 bis 3, worin der Kupferkatalysator Cuprochlorid ist.

5. Verfahren nach einem der Ansprüche 1 bis 4, worin der katalytische Anteil des Kupferkatalysators innerhalb eines Gewichtsbereiches von 0,01 bis 2,0% des Allylhalogenids liegt.

6. Verfahren nach einem der Ansprüche 1 bis 5, worin das Molverhältnis des organischen quaternären Salzes zum Kupferkatalysator 0,01 bis 5,0 beträgt.

7. Verfahren nach einem der Ansprüche 1 bis 6, worin die Isomerisierung während eines Zeitraumes von 0,1 bis 10,0 Stunden durchgeführt wird.

8. Verfahren nach einem der Ansprüche 1 bis 7, bei dem das organische quaternäre Salz ein Gemisch aus Tri(alkyl)methylammoniumchloriden ist, worin der Alkylanteil eine Kette von 8 bis 10 Kohlenstoffatomen umfasst.

9. Verfahren nach einem der Ansprüche 1 bis 8, worin das organische quaternäre Salz ausgewählt ist aus der Gruppe, bestehend aus Methyltrioctylammoniumchlorid, Dimethylbenzylstearylammonium, N,N-Cetylethylmorpholiniumethosulfat, Benzylhydroxyethyl(2)-cocoimidazoliumchlorid, N-Talg-pentamethyl-propandiammoniumdichlorid, Benzyltrioctylammoniumbromid und Propyltriphenylphosphoniumbromid.

10. Verfahren nach einem der Ansprüche 1 bis 9, worin das Allylhalogenid Linalylchlorid ist.

11. Verfahren nach einem der Ansprüche 1 bis 9, worin das Allylhalogenid 3-Chlormethyl-1-buten ist.

**Revendications**

1. Un procédé d'isomérisation d'un halogénure allylique en son isomère allylique qui comprend l'isomérisation de l'halogénure allylique en présence d'une certaine proportion d'un acide halohydrique et d'un catalyseur à base de cuivre, l'amélioration consistant à mettre en œuvre l'isomérisation en présence d'un sel quaternaire organique choisi parmi ceux présentant la formule:

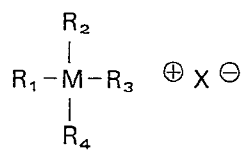

dans laquelle:

X est choisi dans le groupe comprenant les anions minéraux et organiques tels que nitrate, benzoate, phénylacétate, hydroxybenzoate, phénoxyde, hydroxyde, cyanure, nitrite; les chlorure, bromure, iodure, le sulfate de méthyle, le sulfate d'éthyle et analogue étant tout particulièrement préférés;

M représente un atome d'azote, d'arsenic ou de phosphore;

$R_1$, $R_2$, $R_3$ et $R_4$ sont indépendamment choisis parmi les radicaux hydrocarbonés substitués ou non, étant entendu que, lorsqu'un ou les deux radicaux $R_1$ et $R_2$ contiennent de 1 à 4 atomes de carbone, bornes incluses, les autres radicaux R contiennent de 6 à 25 atomes de carbone, ou que $R_1$ et $R_2$ peuvent former ensemble un radical divalent lié à l'atome de M, choisi dans le groupe comprenant les radicaux alkénylènes et alkénylènes substitués par un reste hydrocarboné formant un noyau comportant 5 à 10 atomes de carbone, bornes incluses; ou que $R_1$ et $R_2$ peuvent former ensemble avec l'atome de M auquel ils sont liés un

radical divalent ou monovalent choisi dans les groupes présentant la formule:

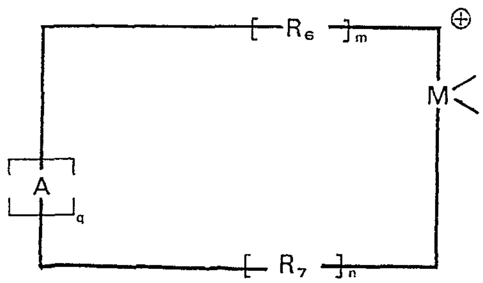

dans laquelle

A représente un atome d'azote, d'oxygène, de soufre, de phosphore et analogue; et

$R_6$ et $R_7$ sont chacun choisis dans le groupe des radicaux alkénylène et alkénylène à substituant hydrocarboné comprenant 1 à 25 atomes de carbone, bornes incluses,

m, n et q sont des nombres entiers, égaux à 0 ou 1 et la somme de m + n est égale à 1 ou 2.

2. Le procédé selon la revendication 1, dans lequel l'halogène est du chlore.

3. Le procédé selon la revendication 1 ou 2, mis en œuvre à une température comprise entre 0° C et 50° C.

4. Le procédé selon l'une quelconque des revendications 1 à 3, dans lequel le catalyseur à base de cuivre est le chlorure cuivreux.

5. Le procédé selon l'une quelconque des revendications 1 à 4, dans lequel la quantité de catalyseur à base de cuivre représente de 0,01 à 2,0% en poids par rapport à l'halogénure allylique.

6. Le procédé selon l'une quelconque des revendications 1 à 5, dans lequel le rapport molaire sel quaternaire organique/catalyseur à base de cuivre est de 0,01 à 5,0.

7. Le procédé selon l'une quelconque des revendications 1 à 6, dans lequel l'isomérisation est mise en œuvre pendant une période de 0,1 à 10,0 heures.

8. Le procédé selon l'une quelconque des revendications 1 à 7, dans lequel le sel quaternaire organique est un mélange de chlorures de tri(alkyl)méthylammonium dans lesquels la partie alkyle comprend une chaîne comprenant 8 à 10 atomes de carbone.

9. Le procédé selon l'une quelconque des revendications 1 à 8, dans lequel le sel quaternaire organique est choisi dans le groupe comprenant chlorure de méthyltrioctylammonium, diméthylbenzylstéarylammonium, éthosulfate de N,N-cétyléthylmorpholinium, chlorure de benzylhydroxyéthyl(2)-cocoimidazolium, dichlorure de N-suif-pentaméthylpropanediammonium, bromure de benzyltrioctylammonium et bromure de propyltriphénylphosphonium.

10. Le procédé selon l'une quelconque des revendications 1 à 9, dans lequel l'halogénure allylique est le chlorure de linalyle.

11. Le procédé selon l'une quelconque des revendications 1 à 9, dans lequel l'halogénure allylique est le 3-chloro-méthyl-1-butène.